(19) **Europäisches Patentamt**
**European Patent Office**
**Office européen des brevets**

(11) **EP 2 067 438 B1**

(12) **EUROPEAN PATENT SPECIFICATION**

(45) Date of publication and mention
of the grant of the patent:
**02.01.2013 Bulletin 2013/01**

(51) Int Cl.:
*A61B 5/021* (2006.01)          *A61B 8/08* (2006.01)
*A61B 7/00* (2006.01)

(21) Application number: **08004112.2**

(22) Date of filing: **05.03.2008**

(54) **Device for and method of noninvasive measurement of parameters of diastolic function of left ventricle during exercice and at rest**

Gerät und Verfahren zur nichtinvasiven Messung von Parametern der diastolischen Funktion des linken Ventrikels bei Beanspruchung und in Ruhe

Dispositif et procédé de mesure non-invasive des paramètres de fonction diastolique de ventricule gauche au cours d'un exercice et au repos

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **04.12.2007 EP 07122262**

(43) Date of publication of application:
**10.06.2009 Bulletin 2009/24**

(73) Proprietor: **Manolas, Jan**
**TT 153 52 Athens (GR)**

(72) Inventor: **Manolas, Jan**
**TT 153 52 Athens (GR)**

(74) Representative: **Beck & Rössig**
**European Patent Attorneys**
**Cuvilliésstrasse 14**
**81679 München (DE)**

(56) References cited:
**US-B2- 7 107 095**

• **MANOLAS J ET AL: "Identification of patients with coronary artery disease by assessing diastolic abnormalities during isometric exercise." CLINICAL CARDIOLOGY NOV 2001, vol. 24, no. 11, November 2001 (2001-11), pages 735-743, XP002519030 ISSN: 0160-9289**

## Description

FIELD OF THE INVENTION

This invention is in cardiology and relates to the detection of heart dysfunction by use of an exercise test in human beings. More specifically, the invention relates to a device and method for noninvasive measurement of parameters of diastolic function of the left ventricle

BACKGROUND OF THE INVENTION

Pressocardiography represents a noninvasive technique which is obtained by means of an external pressure transducer transthoracically, providing the opportunity to measure left ventricular (LV) pressure curve changes in diastole at rest and during short isometric exercise by an operator with minimal medical skills. The inventor, Dr. Manolas, holds a U.S. patent for a device and method of rapid noninvasive measurement of parameters of diastolic function of the left ventricle which was published under US 7,107,095 B2.

Doppler echocardiography is a generally known procedure that uses ultrasound technology to examine the heart by creating an image of it and measuring the speed and direction of blood flow.

Up to now, both techniques are used at Rest and with Exercise separately. Corresponding studies were made by Dr. Manolas and published at the 1st European Diastology Meeting 1996 and at the 1st Annual Meeting of the Heart Failure Society of America 1997 as well as in Clin. Cardiol. 24, 735-743 (2001) "Identification of Patients with Coronary Artery Disease by Assesing Diastolic Abnormities during Isometric Exercise".

As proved in recent years using imaging techniques, the assessment of LV function in early and late diastole is of fundamental clinical significance. LV diastolic dysfunction is the earliest manifestation in the course of most common and life threatening as well as often subclinic myocardial diseases and, in addition, is associated with symptoms and worse prognosis as well as with increased rate of hospitalizations. It should be emphasized that coronary artery disease (CAD), hypertensives and congestive heart failure patients show in >90%, >70% and >55%, respectively, some degree of LV diastolic function abnormalities at rest or/and during effort. Further, it is now proved and widely accepted that the incidence and the prognosis of patients with heart failure and preserved systolic dysfunction do not differ

significantly from that of patients with "classical heart failure" having decreased systolic function of left ventricle. Thus, the LV ejection fraction does not appear to be the single most powerful index of LV function, as it was believed until recently, since the assessment of LV diastolic function is also essential in this clinical setting.

It is widely accepted that intrinsic LV diastolic chamber properties can ideally be assessed using both volumetry and pressure transducer derived measurements. An inappropriate upward shift of the pressure-volume relation can lead to symptoms of congestion independently if this shift is caused by an increased pressure for a given volume or by an increased pressure disproportionate to increased volume. This shift in pressure-volume (PV) relation can occur at rest or particularly during exercise leading to rise in pulmonary wedge pressure and lung congestion. In cases with so-called "pure LV diastolic dysfunction or diastolic heart failure" having not-dilated left ventricle and good systolic function, diastolic alterations occur predominantly in LV pressure curve and much less in LV volume with an isolated upward shift of the PV relation.

Ideally, one should assess both the LVP curve and the LV volume changes in early (Relaxation) and late (Compliance) diastole. However, this accurate and pathophysiologically correct and accurate method consists in the use of simultaneous angiography and catheter tipmanometer derived measurements. Since a "pure LV diastolic dysfunction" is occurring frequently and early, i.e. before systolic or ECG changes or symptoms, in the course of most myocardial diseases (like arterial hypertension, coronary artery disease, diabetes mellitus etc) and is associated with increased mortality and hospitalization rate, its non-invasive assessment is of great clinical importance. Hitherto, only volumetric techniques are widely used for assessing LV diastolic function and almost exclusively at rest, being mostly time-consuming, expertise needing and rather impractical as routine tools for assessing LV filling pressures. Especially, in patients with good contracting and not-enlarged left ventricle most volumetric measurements are rather debatable.

On the other hand, all investigators exploring the accuracy of volumetric techniques for assessing diastolic function still use LVP and left atrial (LA) pressure recordings as the gold standard. Thus, it is generally accepted that pressure transducer derived measurements reflect more accurately the LV diastolic alterations. Further, conclusive evidence exists that the earliest manifestation of ischemia is diastolic dysfunction -initially relaxation and later end-diastolic abnormalities, preceding both the ECG and the systolic abnormalities on the onset of every ischemic episode.

The assessment of diastolic behavior of LVP curve during dynamic or isometric exercise has been found of clinical significance. Above conclusive evidence exists that LVP behavior during isometric or dynamic exercise differs greatly between healthy and myocardial disease patients as well as among the latter. Especially, the LVP behavior during isometric handgrip (HG) exercise showed dramatic alterations in the presence of induced ischemia within few minutes, whereas volumetric indices showed significantly less pronounced changes. These HG-induced LVP changes -above LVEDP rise-have been proved that they are of ischemic origin being associated with wall motion abnormalities in angiography . A. P. Flessas et al., "Effects of isometric exercise on the end-diastolic pressure, volumes, and function of the left ventricle in man", Circulation, Vol. 53, p. 839-847 (1976) Further, HG-induced ischemia has been shown to be associated with worse prognosis due to severe endothelial dysfunction even in absence of obstructive CAD . B. D. Johnson et al., "Persistent chest pain predicts cardiovascular events in women without obstructive coronary artery disease: results from the NIH-NHLBI-sponsored Women's Ischaemia Syndrome Evaluation (WISE) study", European Heart Journal, Vol. 27, No. 12, p. 1408-1415 (2006)

In order to assess both "pure LV diastolic dysfunction or diastolic heart failure" as well as LVP diastolic behavior with exercise, volumetry - including Doppler echocardiography - alone does not appear sufficient enough; the use of a pressure transducer being more than essential in clinical practice in this setting.

Based initially on published catheterization data and subsequently in his own clinical experience with >7.000 patients and published data using an external optimal pressure transducer directly applied on the thoracic wall (=tipmanometer), the inventor has introduced, analyzed and recognized the clinical value of the "diastolic stress test" concept based on published data derived from A) invasive LVP recordings by means of catheter tipmanometer, and B) non-invasive Tipmanometer-derived recordings (=pressocardiograms).

*I. Invasive Assessment of LV diastolic function at Rest and during Exercise*

It is widely accepted that invasively obtained LVP curves by means of micromanometer represent still today the gold standard for assessing accurately LV diastolic function. The most common diastolic indices are those of the level of LV filling pressure (Compliance) and LV pressure curve fall (Relaxation), both being at best assessed from high-fidelity LV pressure recordings in catheterization laboratory.

In early diastole, the most commonly used LVP fall variables are the time constant of relaxation (Tau), the total LV relaxation time and min dP/dt.

In late diastole, the most reliable are end-diastolic parameters; namely, LV end-diastolic pressure (LVEDP), pre-atrial or pre-A wave as well as peak LA pressures.

It has been shown using micromanometer measurements from internal LVP recordings that the changes of some diastolic variables during isometric HG exercise, especially at the end-diastole, are characteristic for the presence of some myocardial disease states. Whereas patients with normal or nearly normal LV function show no or only minimal diastolic changes during isometric exercise, patients with CAD show mostly dramatic alterations and particularly rise in LVEDP. It should be noted that this rapidly occurring and high elevation of LVEDP is also present during other exercise modalities like dynamic exercise by means of bicycle or treadmill.

By analysis of published heart catheterization data as well as based on his own prior clinical experience in cath lab of University of Zurich, it was realized by the inventor for the first time that significant diastolic alterations in LVP curve during exercise in CAD patients represent a characteristic sign of ischemia. The term "ischemic diastolic response" has been introduced characterizing all these typical LVP curve changes. The inventor also recognized that the assessment of this "ischemic diastolic response" in daily clinical practice could become of potentially great importance. It should be also noted that the corresponding volumetric measurements are less dramatic having a minor magnitude of changes in presence of induced ischemia.

It has also been proved in human beings U. Sigwart et al., "Left atrial function in acute transient left ventricular ischemia produced during percutaneous transluminal coronary angioplasty of the left anterior descending coronary artery", The American Journal of Cardiology, Vol. 65, No. 5, p. 282-286 (1990)) that during ischemia induced by occlusion of a coronary vessel during angioplasty in the left anterior coronary vessel, an extreme prolongation of total relaxation time of the left ventricle occurs within 3-5 sec and is followed after 15-25 sec by a dramatic rise in LVEDP and LA pressures which are reaching a more than 150% of the base line values.

Based on heart catheterization data derived from high-fidelity LVP recordings by means of micromanometers and exact early and late diastolic measurements, it has further been shown that in the presence of ischemia the diastolic abnormalities of LVP curve are much more pronounced than those of Volumetry -either in diastole or systole (including LV ejection fraction).

In last decade, the clinical value of applying this "diastolic stress test" concept for detecting an "ischemic diastolic response" has been proved by the inventor (published data and clinical experience) using high-fidelity pressocardiographic recordings by means of external tipmanometer during HG in a large cohort of > 7.000 healthy subjects and patients.

*II. Non-invasive Assessment of LV diastolic function*

According to the mentioned data, it is logical to assume that the assessment of LV filling pressures using non-invasive volumetric or pressure transducer-derived measurements by Diastolic stress echo- or pressocardiography (presso test) in everyday practice at rest and during short exercise, could be of fundamental clinical importance.

Consequently, it is expected that the application of a simple small and user friendly device, at best portable or handheld, for obtaining a non-invasive "diastolic stress test" assessing simultaneously the mentioned diastolic LVP curve as well as the volume and velocity alterations within the left ventricle in everyday practice, would represent a very useful daily tool leading us probably to an easier and earlier identification of patients with subclinic CAD before the onset of symptoms or systolic dysfunction (assessed by echocardiogram) or electrical changes (in ECG).

This combined echo-presso device could help greatly in outpatient facilities for sorting out patients with atypical symptoms (chest pain or dyspnea or discomfort). Recently, there are published data showing that presso test can be useful in evaluating patients with or without symptoms and suspected CAD (Manolas et al. 2001); more recently, diastolic stress echocardiography has been shown to be useful in evaluating patients with dyspnea . M. I. Burgess et al., "Diastolic Stress Echocardiography: Hemodynamic Validation and Clinical Significance of Estimation of Ventricular Filling Pressure With Exercise", Journal of the American College of Cardiology, Vol. 47, No. 9, p. 1891-1900 (2006)

It should be emphasized that the evaluation of all non-invasive volumetric -including Doppler echocardiographic- diastolic indices is done by comparing them with pressure transducer derived variables; this fact proving that the latter are much more accurate and sensitive indices of diastolic functional alterations, especially in good contracting and not-dilated hearts.

It is expected, that a wide application of a combined PV assessing instrumentation would enable a more accurate assessment of LV diastolic behavior in clinical practice and probably would result in an improved secondary prevention by leading us to an earlier onset of preventive and/or therapeutic strategies.

Most importantly, an isometric HG exercise of 2 min duration and 40% of maximal voluntary contraction represents a low level stress to the human left ventricle and all alterations are quickly reversible. In addition, this quick and slight HG can be performed on the bed by almost all patients using the one hand and is advantageous for assessing in an optimal way diastolic variables because of less increase in heart rate. Thus, compared to the troublesome and often inconvenient dynamic (bicycle or treadmill) or pharmacological (adenosine or dobutamine) stress testing modalities, the use of a low level and short HG exercise represents a faster, safer and more convenient exercise modality providing also a more optimal measurement of diastolic indexes.

Basically, there are mainly two non-invasive methods for assessing LV diastolic function changes in daily routine practice: the previously widely applied "apex" or pressocardiography using an external optimal pressure transducer (=tipmanometer) and the currently widely applied Doppler-echocardiography (mainly TDI) assessing volume and velocity changes. The former reflects in time, slope and relative amplitude LVP curve changes and the latter abnormalities in early and late flow velocities.

The hitherto widely used current methods of measuring diastolic LV function are almost exclusively imaging approaches - like nuclear ventriculography and modem Doppler-techniques (TDI), which are not applied routinely in every patient and often not out-of-hospitals.

On the other hand, although not widely used presso test remains the only non-invasive technique using an external optimal pressure transducer directly applied on the thoracic wall for obtaining "non-invasive LVP recordings", as Prof. Hugo Kesteloot -Leuven, Belgium- was emphasizing in his lectures, both at rest and during HG. This simple effective tool is obtained as initial screening tool by any not skilled personnel in every office by non-experts.

*a. Non-invasive Assessment of LV diastolic dysfunction by external pressure transducer*

At the end of 60s and early 70s, attempts have been made to obtain optimal recordings from the point of maximal pulse of the heart beat - referred as "Apexcardiography"- but have been handicapped mainly because of lack of an advanced instrumentation. Further, the assessment of "apexcardiography" was not reached a high level including an automated evaluation of exactly defined diastolic indices as well as of exactly defined patterns, types and forms of isometric exercise-induced changes in diastolic profile.

All these previous attempts at obtaining and evaluating (apex)pressocardiographic waveforms have been handicapped by the lack of advanced instrumentation, signal processing and pattern recognition techniques. Unfortunately, the initially used "pulse transducers" had a mostly unknown or too low time constant (<3 sec) using an AC input and piezoelectric recording systems with air transmission by means of air filled tubes. These earliest versions of "displacement cardiography" were designed as ballistocardiography and kinetocardiography being rather qualitatively than quantitatively evaluated and mostly also in relation to the phonocardiographic recordings. It should be mentioned that apexcardiograms (0-50 cps) have significantly higher amplitudes than phonocardiograms (100-500 cps). These "apex tracings" have been obtained mostly at rest and primarily used for an improved interpretation of heart sound and murmurs. However, already in earlier years the significance of measuring the relative A wave amplitude and less also of the relaxation time of the "apex tracing". Nevertheless, even using these technologically not optimal apical recordings after dynamic exercise, the widely applied "exercise apexcardiography" has become a useful daily tool in evaluating CAD patients in late 60s and 70s. However, the diastolic waves of these "recordings of the apical pulse" after dynamic exercise could not be exactly evaluated due to rapid heart rate and above inadequate "pulse transducers" and pre-amplifiers. Namely, both amplitude and above relaxation time indices are greatly influenced by the mode of pick up device and time constant of the pressure transducer. In addition, the air- or fluid-filled tubes which were used in 60s and early 70s for transmission of the pulse signal and transducers with too short time constant were leading to distorted tracings, reflecting much less accurate LVP curve changes.

Nevertheless, most precordial (and above apexcardiography) and other (carotid pulse and jugular vein tracings) mechanocardiographic techniques have been all used successfully in the daily clinical practice for many years in the daily practice as helpful routine tools. Out of all these mechanocardiographic techniques only arterial pulse recordings have been re-evaluated and started again to become routine techniques. They are now widely applied as "arterial pulse wave" tracings for assessing arterial pulse wave velocity and arterial elasticity or compliance, receiving widespread acceptance.

In the end of 70s, however, several investigators have used for the first time optimal external pressure transducers with infinite time constant being applied directly on the thoracic wall (=tipmanometer) without any fluid or air transmission by means of tubes; the latter leading to significant distortions of the tracings.

By comparing simultaneously obtained internal and external high-fidelity tracings by means of catheter and external tipmanometers, Jan Manolas and other cardiologists in University of Zurich, proved for the first time in human beings the significant correlation between relative A wave and Total Relaxation time variables with LVEDP and min dP/dt derived from LV pressure curve, respectively. Other investigators have proved that "apexcardiographic" and LVP recordings are very similar and often identical in time course, slope and relative amplitude. Thus, the term "pressocardiograms" reflects more accurate the true source and nature of these external tracings which rather represent "non-invasive LVP curve recordings" than anything else (Prof. Hugo Kesteloot, Belgium).

Hitherto, pressocardiography represents the only noninvasive technique being obtained by means of an optimal external pressure transducer transthoracically, providing the opportunity to measure accurately and unidirectionally LV pressure curve changes in diastole at rest and during short isometric exercise (called presso test) by an operator with minimal medical skills.

In last years, using external tipmanometer for obtaining high-fidelity pressocardiographic recordings, we have proved that the non-invasive assessment of differences in LV diastolic behavior using pressocardiography during HG exercise enables the differentiation of myocardial disease patients. It is expected that a wider application of the introduced by the inventor "Diastolic stress test" and "Differentialforms" concepts, could become of great clinical importance in the daily routine practice. The application of the "diastolic stress test" concept using external tipmanometer and short low level isometric exercise (presso test), represents the opportunity to measure diastolic performance of the human left ventricle and detect potentially life-threatening abnormal heart conditions in an early phase before development of symptoms or ECG or other signs of myocardial disease states and especially CAD.

Thus, presso test represents a simple and not-expertise needing as well as fast and safe diagnostic tool. Although not yet widely applied, could soon become an ideal initial screening test in the hands of every practitioner including family and general practitioners.

The inventor has found for the first time that a very similar to LVP curve behavior dramatic pattern of changes in early and/or late diastole during isometric exercise occur also using the presso test in patients with known or subsequently proved CAD with inducible myocardial ischemia, whereas healthy subjects show no or minimal changes within exactly defined normal limits. Thus, it appears that diastolic changes in pressocardiographic curve during HG exercise are also unidirectional and very similar to those found internally in LVP curve during catheterization. These published data show how useful would be an adding of pressocardiography both in non-expert hands of every practitioner and in the Doppler-echo devices for an even more precise assessment of LVP curve in diastole at rest and during ischemic diastolic response and evaluation of patients with diastolic heart failure.

As previously described, a device for isometric HG exercise by the patient is used, e.g. a balloon dynamometer, which should be squeezed at approximately 40% of the maximal voluntary contraction during 2 min. At rest during and after HG exercise the following variables are calculated semi-automatically by means of a normalized pressocardiographic and phonocardiographic digital signal:

- A/H = the relative A wave height to total systolic-diastolic excursion of pressocardiogram
- A/D = the relative A wave to total diastolic height of pressocardiogram
- TORET = the Total pressocardiographic relaxation Time from begin of aortic component of the second heart sound to the protodiastolic nadir of pressocardiographic curve or O point.
- TORETI = the Total pressocardiographic Relaxation Time Index, which is given by the following formula:

$$\text{TORETI} = \sqrt{A2 - C} / \text{TORET}$$

where A2 is the onset of the aortic component of the second heart sound in the phonocardiogram and C the onset of the pressocardiographic systolic upstroke. TORETI represents a modified Bazett formula, which has been used to correct temporal variables for heart rate; whereas the duration of diastolic phase is being used instead of R-R interval.

*b. Non-invasive assessment of LV diastolic dysfunction by Doppler echocardiography*

All Doppler echocardiographic techniques are volumetric methods providing ultrasonic dynamic images of the heart like transmitral flow velocities, pulmonary venous flow assessment, acoustic quantification, flow propagation assessment and the most recently introduced Tissue Doppler Imaging and Strain-Rate Imaging.

In last decades, numerous or rather myriads indices of LV diastolic function have been introduced using multiple Doppler echocardiographic methods for estimating the filling profile and above the LV filling pressures. Most of these indices have never been widely applied in clinical practice and several of them have been abandoned before even defining their normal limits. For many years, the ratio of early to late transmitral velocity (E/A) has been accepted as the standard diastolic index and is still widely used by most cardiologists. In patients with clinical signs of heart failure, the problem of a "pseudonormalization" of E/A presented a profound and frustrating limitation; whereas a "restrictive profile" have become a sensitive index of elevated LV filling pressures predominantly in the presence of enlarged bad contracting left ventricle in clinical practice. An E/A <1 represented an indicator of abnormal relaxation with decreased early filling of the left ventricle, whereas an E/A » 1 an indicator of decreased LV distensibility, especially in patients with dilated and bad contracting left ventricle. In addition, a shortened deceleration time of the transmitral early flow velocity (DT) was also used as an index of rised LV filling pressures.

Subsequently, the adding of pulmonary venous flow variables have subsequently helped echocardiologists greatly in discriminating better the presence of "pseudonormalization" of velocities profile.

More recently, tissue Doppler imaging (TDI) has been introduced as a more reliable method to evaluate diastolic dysfunction by measuring mitral annulus velocity during diastole; the most widely accepted and clinically applied diastolic index of TDI is the ratio of peak early mitral inflow velocity E and the analogous E' (E/E'); the latter being measured from the apical 4-chamber view with sample volume placed at the septal corner of the mitral annulus. It has been shown that LV filling pressures can often be estimated reliably by this E/E' ratio.

In last years, researchers realized that symptoms, mainly dyspnea, and a decreased exercise capacity in patients with "pure diastolic dysfunction and diastolic heart failure", occur predominantly during exertion because of rising LV filling pressures. More recently, inspired and taking obviously into account the recently introduced by Dr. Manolas "Diastolic stress test" concept, some researchers have finally realized the potential great value of assessing LV filling pressures with exercise and started to explore the changes of the mentioned TDI indices after exercise and introduced the "diastolic stress echocardiography". However, these studies aimed to potentially improve the evaluation of patients with dyspnea on effort and did not attempt to assess ischemia-induced diastolic dysfunction. The researchers found that whereas a great part of these patients with dyspnea on effort had a normal E/E' at rest, but an >30% of them showed an increased E/E' with exercise.

However, according to the published data of this novel "diastolic stress echocardiography" most of patients with CAD, there was a great difference between the magnitude of LVEDP rise and E/E' increase with exercise; the former being >140% and the latter only 33% from the baseline; interestingly, also this dramatic LVEDP rise in CAD patients is in accordance to the "diastolic stress test & ischemic diastolic response" concepts of Dr. Manolas demonstrating the typical dramatic LVEDP rise in CAD patients due to compliance decrease. However, the ischemic diastolic response of LVP curves can, unfortunately, only partly be assessed by pure volumetric measurements -even using the best and most widely accepted and applied TDI index in last years. In CAD patients, the pressocardiographic A/H shows in an average increase during HG of 70-130% G, whereas the E/E' an only <40% increase from baseline.

Thus, it appears that pressure transducer-derived indexes are essential for detecting the high-amplitude diastolic changes in CAD patients. Consequently, it is expected that by adding pressocardiography to echocardiographic recordings, there will be a significantly increased sensitivity and specific in assessing the great magnitude of LVP diastolic changes during exercise in CAD patients with inducible ischemia.

That pressure transducer-derived measurements represent a more accurate technique is proved also by the fact that LVP diastolic indices are used extensively -if not exclusively- by all researchers of volumetric techniques as the absolute gold standard.

SUMMARY OF THE PRESENT INVENTION

This patent application addresses the previous difficulties in assessing non-invasively LV diastolic dysfunction at rest and above with exercise by combining two pathophysiologically completely different non-invasive diastolic techniques: a widely used volumetric technique, i.e. Doppler echocardiography with the only external pressure transducer using method, i.e. pressocardiography.

It is logically expected that this combination will improve significantly the diagnostic power and practically usefulness of the "diastolic stress test" as an initial screening tool combining a volume and a pressure assessing technique. Most importantly, by developing a novel combined velocity-pressure device, this physiologically more correct mode in a similar way like invasive cardiologists use P and V variables for assessing intrinsic LV diastolic properties and performance, could lead us to an improved non-invasive estimation of LV filling pressures at rest and above the LVP diastolic behavior during exercise enabling a more sensitive detection of myocardial ischemia and potentially differentiation of myocardial disease states. In addition, the application of a fast, convenient and safe low level modality of exercise testing by means of short isometric HG exercise test, would increase the practicability of a such "Combined Diastolic Echo-Presso Stress Test" in the daily routine practice.

The invention provides a device for and method of noninvasive measurement of parameters of diastolic function of left ventricle and automated evaluation of the measured profile of LV function at rest and with exercise using combined presso- and echocardiography as indicated in the claims.

The technique consists in obtaining non-invasively from the chest wall recordings by means of both an optimal external pressure transducer (so-called pressocardiograms) and of an echo probe (Doppler-echocardiograms) in order to detect diastolic dysfunction of the left ventricle at rest and during exercise -especially a low level short exercise.

The present inventor recognized the expected great advantages of adding the by him introduced Presso Test technology to the widely accepted Doppler echocardiography as an essential methodical and instrumental improvement.

A new definition of the positive/negative result and evaluation of types and a revised formulation of diastolic patterns by newly defined combined diastolic indices, could help greatly in routine practice.

The introduction of new definitions of new combined diastolic indices, represents a significant and clinically important improvement of both Doppler echo- and pressocardiography, since the new system is providing information about both sides of diastole, namely pressure and volume changes within the left ventricle; enabling potentially an improved information about intrinsic LV diastolic properties.

This invention could become a very useful novel diagnostic modality in outpatient facilities - especially in chest pain units (CPUs) for shorting out of patients with suspected myocardial dysfunction - above ischemia and in office-based practice for detecting silent or atypical myocardial disease states as well as in sports centers for screening athletes for the presence of subclinic or atypical cardiomyopathies.

In particular, new combined diastolic indices are introduced by the inventor as indicated in claim 10.

The inventor has recognized that the mentioned problem of non-invasive assessment of LV diastolic function changes at rest and with exercise could technologically at best be solved by the only pathophysiologically correct way, namely by applying a "combined pressure-echo transducer" for obtaining simultaneously mitral inflow and annulus velocities as well as pressocardiograms. Additionally, the inventor recognized that the use of a short, low level isometric HG as a convenient and safe exercise modality offers great advantages for both the patient and the examining physician overcoming the potentially dangerous and troublesome application of dynamic or pharmacological stress testing; whereas also the latter 2 modes could provide in a more sophisticated and time-consuming way excellent information.

It was also recognized that in addition to the previously introduced in literature and further developed by the present inventor presso test technique as well as the recently introduced diastolic stress echocardiography technique, a "combined diastolic stress echo- and pressocardiography" using either dynamic or pharmacologic exercise or at best HG exercise would significantly enhance the diagnostic power of each single of these techniques and especially diminish the limitations of applying an isolated volumetric method for estimating LVP behavior with exercise.

The development of a combined echo-presso sensor unit would enable a routine application of this novel tool initially - just after the rest-ECG and rest-echo - as an initial screening stress test, before Dobutamine or treadmill exercise tests using the classical exercise echocardiography or nuclear tests, for an early identification and potentially differentiation of myocardial disease patients resulting in a faster and safer cardiac evaluation in daily practice for an improved prevention of acute myocardial infarction or sudden death.

A new device comprises: an external transthoracically applicable pressure transducer - at best a microtipmanometer fixed in a tube - for obtaining an optimal high-fidelity non-invasive LV pressure curve and an echo probe, both being connected as one echo-presso transducer; these transducers being applicable either separately or as one combined transducer. Further, this unit could be combined or not with an external heart sounds microphone and a device for performing an isometric HG exercise. For obtaining optimal pressocardiograms, the pressure transducer should have a flat response in a range from 0 to 100 cps and at least from 0 to 50 cps. Further, a unit for measuring and semi-automatic calculating parameter of early and late diastole derived from the transthoracically recorded LVP curve (pressocardiogram) and a computer-based automated calculation of pressocardiographic indices as well as combined echo-presso indices invented by Dr. Manolas. In future, a differentiation of Ischemic vs Non-ischemic behavior could be developed according to exactly defined patterns. For the first time, cardiac patients are evaluated and classified based on a combined non-invasive pressure-volume estimation at rest and during exercise by means of external echo- and pressocardiographic instrumentation.

The phonocardiogram could also be obtained by filtering the higher (frequencies 100-1000 cps) from the pressure transducer.

An automated interpretation by a portable instrument (handheld echo equipment with pressure and heart sounds channels) would improve the necessary visual and empirical interpretation of the test result enhancing considerably the practical clinical applicability of this combined "diastolic echo-presso stress testing".

Additionally, the use of a short, low level, convenient and safe mode of exercise like isometric handgrip exercise shows very advantageous; this simple stress test having the additional advantage of being associated with only minor heart rate increase without inconvenient treadmill or bicycle or pharmacologic test and with the patient in recumbent position. However, dynamic or pharmacological stress testing could also be used in future using this "combined echo-pressocardiography" modality adding significant clinical information in specific clinical settings.

This combined echo-presso stress test using short-low level HG exercise could enable a faster, more convenient and easier novel mode of initial screening asymptomatic patients with subclinic myocardial diseases beyond the information given by ECG or rest echocardiography. This novel stress test modality could contribute significantly in sorting out patients in Outpatient facilities - including CPUs - when applied just after ECG and before other more sophisticated, time consuming and costly diagnostic techniques.

As a tool of early secondary prevention could not only become useful for preventing those patients with "silent" myocardial diseases who remain unidentified till their first dramatic acute event or even sudden death but also for detecting or ruling out the presence of acute ischemia in patients with suspected unstable angina.

A potential future discrimination between Ischemic and Non-ischemic according to exact criteria of an "ischemic diastolic response" as well as of "differentialforms" based on combined Doppler echo- and pressocardiographic indexes could enable an improved and practically important classification of patients with suspected or unknown myocardial diseases in daily practice.

The new device comprises: an external transthoracically applicable unit including both a pressure transducer for obtaining a non-invasive LVP mirroring curve and an echo probe for obtaining Doppler echocardiographic measurement, whereas these transducers could also be used separately, an external heart sounds microphone, which could be used separately or included in the combined echo-presso probe, a device for performing an isometric or dynamic exercise; further, a Doppler echocardiographic equipment, a pre-amplifier and a unit for measuring and semi-automatic calculating parameter of early and late diastole derived from the transthoracically recorded LVP curve (pressocardiogram) and Doppler echocardiogram including TDI, and potentially a computer-based automated categorization of "diastolic patterns" or even "diastolic differentialforms" with inclusion of Doppler indices.

The introduction of new definitions and new combined diastolic indices represents a significant and clinically important improvement of both Doppler echo- and pressocardiography, since the new system is providing information about both sides of diastole, namely pressure and volume changes within the left ventricle.

Additionally, this invention could also become a very useful novel diagnostic modality in sports centers for screening athletes for the presence of subclinic or atypical cardiomyopathies.

The inventor has recognized the need of combining Presso and Echo technology for a more accurate assessment of pressure and volume indices of LV diastolic function both at rest and during exercise in the daily practice, which requires an improved instrumentation by means of a combined presso-echo transducer and a computer-based automation of an instrument to produce an automated categorization of the following parameters:

> a) Detection of at least one combined presso-echo parameter which is in a range of a in future exactly defined as abnormal or pathologic;
> b) Discrimination between Early (Relaxation) and Late (Compliance) as well as Mixed (relaxation-compliance) or Global diastolic abnormalities
> c) Potential discrimination between Ischemic and Non-ischemic diastolic reaction with exercise
> d) Potential discrimination of Differentialforms

A further classification consists in separating early and late diastolic abnormalities by defining the "diastolic types":

> a) Early diastolic abnormality (Relaxation or R-type)
> b) Late diastolic abnormality (Compliance or C-type), and
> c) Both C- and R- types are simultaneously present in one single beat (mixed or RC-type)
> d) Global diastolic dysfunction

More particularly, the present invention is disclosed for discriminating myocardial disease patients with exercise-inducible ischemia from those without inducible ischemia; this separation helping potentially greatly in decision making about admission in the hospital and/or indication for other more sophisticated stress tests or performing a CT or MRI or coronary angiography :

> a) An "Ischemic diastolic response" being defined by a significant exercise-induced rise in LV end-diastolic pressure or abnormal one or more of the combined compliance indices 1-4 and 13, 14 and this alteration is >40% (range: 40-80%) of the baseline value at rest or/and abnormal relaxation defined by the presence of one or more of the combined relaxation indices 5-8; this alteration being > 20% (range 20-40%) of baseline.
> b) A "Non-ischemic" diastolic reaction being defined by an abnormal/positive stress test result in absence the of the ischemic criteria

A further classification consists in separating the following differentialforms:

> a) "Hypertensive" differentialform with only slight changes during exercise,
> b) "Coronary" differentialform with dramatic and moderate to severe ischemic diastolic reaction and a deterioration of type with exercise (Normal → R-type → RC-type → C type.

c) "Cardiomyopathic" differentialform with severe prolongation of TORET or/and decrease of E wave and/or moderate to severe compliance indices already at rest and non-ischemic pattern with exercise

d) "Congestive" differentialform with moderate to severe end-diastolic abnormalities already at rest and slight changes with exercise

BRIEF DESCRIPTION OF THE DRAWINGS

The following drawings and description of the illustrated embodiments disclose preferred constructions of the present invention in which the above advantages and features will be readily understood. In the drawings:

FIG. 1    shows a schematic view of an apparatus according to an embodiment of the present invention for calculating and automated evaluation of parameters of the diastolic function of the left ventricle;

FIG. 2    shows a second embodiment of the apparatus;

FIG. 3    shows a third embodiment of the apparatus;

FIG. 4    shows a combined echo-presso transducer; and

FIG. 5    shows a graphical illustration of a typical pressocardiographic curve.

DETAILED DESCRIPTION OF THE INVENTION

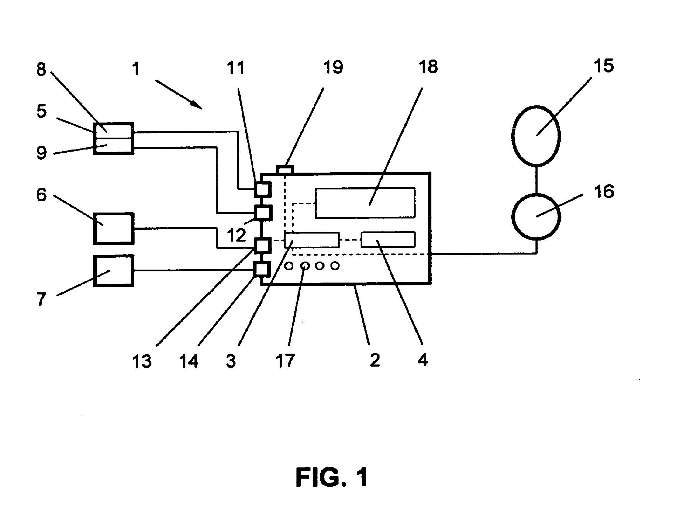

Figure 1 shows a graphic illustration of an apparatus I for a combined "Diastolic Stress Echo-Pressocardiographic Test" analysis and particularly for determining and calculating parameters of diastolic function of the left ventricle comprising a calculation unit in a casing 2 which includes a control device 3 and an interpretation unit 4. The interpretation unit 4 comprises means for processing pressocardiographic signals as well as means for processing echo signals. It may also comprise means for processing phono signals and further means for processing ECG signals.

Further, casing 2 is connected to the following pick up units: a combined echo-pressure transducer 5 for obtaining a tracing which mirrors left ventricular pressure, a heart sounds (phono) microphone 6 for recording the second heart sound and, optionally, an electrical recorder 7 for recording ECG. Apparatus 1 includes also a device 15 for allowing the patient to perform an isometric exercise.

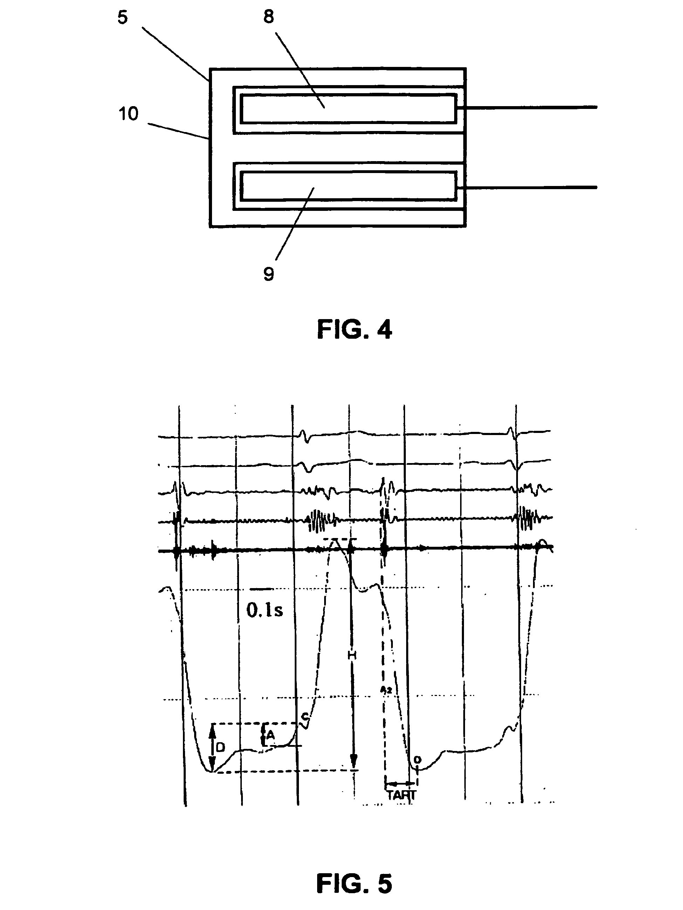

The new combined echo-pressure transducer 5 is shown in Figure 4. The echo-pressure transducer 5 is adapted for being applied on the thoracic wall for obtaining simultaneously Doppler-echocardiograms and pressocardiograms. An echo probe 8 and a pressure sensor 9 are received in a common housing 10. The echo probe 8 and a pressure sensor 9 are connected with separate input ports 11 and 12 of the control device 3 and calculation and interpretation unit 4 at the casing 2 of the calculation unit. Further input ports 13 and 14 can be provided for the microphone 6 and the electrical recorder 7. However, in a basic configuration of a portable unit one can dispense with the additional port 14 of the electrical recorder.

The echo probe 8 and/or the pressure sensor 9 are detachably mounted on the housing 10, e.g. by snap-in connectors, so that each sensor 8 and 9 can be used separately, if required.

It is also possible to combine parts of the housing 10 with each sensor 8 and 9, with both parts of the housing 10 being detachably connectable with each other.

By including both types of sensors in a common housing 10 it is possible to obtain echo- and presso-signals from the same location. Moreover, the handling will be simple and the risk of operational faults and malfunction is reduced.

Control device 3 controls detection and processing of the parameters measured and data output; it also includes a program for guiding a user. The interpretation unit 4 is connected with or implemented in control device 3. Both form an external utilization unit which determines and calculates characteristic diastolic parameters derived from an echocardiogram, a pressocardiogram and a phonocardiogram at rest, during and after exercise as well as combined echo-presso diastolic indices which will be described below. The interpretation unit 4 converts each cardiogram into a digital waveform in the time domain and automatically categorizes the data measured based on categorization criteria stored in the device 1. Storage means store the data from the echo-pressure transducer 5, microphone 6 and electrical recorder 7 during measurement over a predetermined interval. Diastolic echo- and pressocardiographic variables can also be categorized separately.

The non-invasively obtained pressure curves at rest and during isometric HG exercise are termed Presso Test. Using the signal processor of the interpretation unit 4 the diastolic parameters, which will be more fully described hereinafter, will be estimated in all three steps of exercise (rest, during and after exercise). This isometric exercise is preferably performed manually with the one hand, which can be applied to assess the behavior of diastolic parameters with exercise.

Alternatively, whereas dynamic exercise could also be used, it is less valuable for assessing the diastolic behavior of the left ventricle because of a much greater increase in heart rate resulting in a fusion of the diastolic waves.

The non-invasively external echo-pressure transducer 5 is placed on the thoracic wall of the patient over the site of maximal impulse of the heart beat being fixed on the thorax using an elastic strap or used separately or held by hand, if needed. The echo-pressure transducer 5 should have a time constant of at least 1 sec; however, it should be preferred a time constant >3 sec and at best an infinite time constant being similar to the invasively used microtipmanometer. Using this physically correct echo-pressure transducer, one can record high-fidelity pressocardiographic tracings, i.e. without distortions in time and amplitude of the pressocardiograms. The echo-pressure transducer is connected to a pre-amplifier, which increases the signal level; signals of transmitral or pulmonary flow velocities or TDI can be obtained simultaneously with pressocardiograms . The amplified signals are filtered and transmitted to a data acquisition module in which the individual signals are converted in digital format in an already known mode. An example of a similar signal processing system to process the digitized waveform is described in the DE 197 40 931 C2, which is hereby included in the present invention.

The first time derivative dA/dt of the echo- and pressocardiographic curves will be simultaneously obtained either automated or manually calculated from the primary echo and pressure curve. The time constant of the computer for calculating dA/dt was 0.8 - 1.3 ms.

Furthermore, simultaneously with the echo-pressure transducer 5, the separate microphone 6 will also be fixed 5-10 cm medial of the point of the echo-pressure transducer 5; the former being also fixed on the thorax using the same or a separate elastic strap. This microphone 6 is used for recording the heart sounds and particularly a) the onset of the aortic component of the second heart sound for assessing the relaxation temporal parameters, and b) the changes of the atrial or fourth heart sound (S4) or third heart sound (S3) during exercise or c) mitral valve regurgitation due to exercise-induced ischemia.

A manually used device 15 for performing isometric exercise is at best a balloon manometer 16 with a suitable display unit for visualization of the level of the exerted pressure. The echo-presso examination is performed with the patient lying on the bed in the left lateral decubitus position and at the end of normal expiration without closing his mouth (for avoiding Valsalva). In contrast to echocardiography, technically satisfactory pressocardiograms can usually not be obtained in the supine position or during quiet breathing or both; whereas he is advised to squeeze the balloon with the one or both hands at 40% of his maximal voluntary contraction and sustaining his grip for 2 min. The pressure level is displayed at manometer 16, which is connected to control device 3, whereas the level of exerted pressure can be regulated by an automated signal processor. The pressure within the balloon ranged in most subjects between 0.20 and 0.50 kg/cm$^2$. It has been shown that the sustained handgrip on this lower level is sufficient enough for producing cardiac function changes, above in pressure and pressocardiographic function parameters in early and/or late diastole. As variation of this procedure using a balloon dynamometer, several other devices may be used for performing isometric exercise and alternative signal processing systems, e.g. a separated use of balloon dynamometer and the control device 3.

At rest and during the entire duration of - particularly at 30 sec, 60 sec, 90 sec and 120 sec- as well as after isometric exercise, echocardiograms, pressocardiograms and optionally phonocardiographic as well as eventually ECG tracings are when created outputted to a suitable display which may be a screen and/or a printed display. Since the level of isometric stress to the heart is low, no advance events or complications like serious arrhythmias, myocardial infarctions or deaths have been hitherto reported; this representing a great advantage compared to other modes of stress testing like dynamic or pharmacological stress.

For the purpose of operation, the apparatus 1 comprises input devices 17 and a display device 18 provided on the casing 2 for monitoring a stress test. The display device 11 may also show the parameters measured and may, in the event of positivity criteria or an automatic classification of severity of diastolic dysfunction and Types or a differential diagnosis, issue a corresponding message. The casing 2 further comprises a data interface 19 for connection with a handheld computer, notebook computer or PC or a printer in order to transmit parameters measured, diagrams, error messages, etc.

In a modification of the preferred embodiment the control device 3 and/or interpretation unit 4 is an external computer. Thus, it would be possible to use the apparatus 1 only for collecting data whereas interpretation is performed later. It is also possible to integrate all three units in a pocket device, e.g. a PDA.

Preferably, the casing 2 is a handheld portable unit, which forms together with the sensors 5, 6 and 7 and the exercise device 15 an independent measuring apparatus.

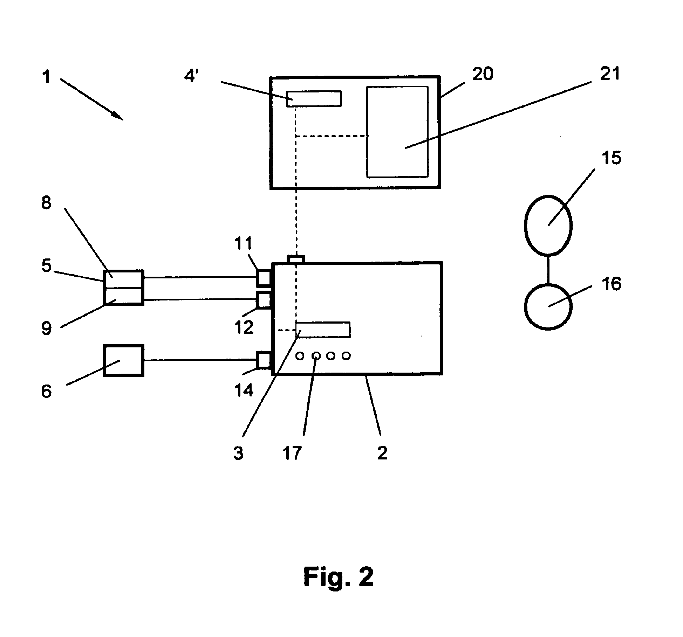

The second embodiment in Fig. 2 shows another configuration, wherein the control device 3 is arranged in the casing 2 and the interpretation device 4' is an external computer 20 which is connected with an external display device 21.

The embodiment in Fig. 2 uses the following pick up units, namely the combined echo-pressure transducer 5 for obtaining a tracing which mirrors left ventricular pressure, and the heart sounds (phono) microphone 6 for recording the second heart. The apparatus 1 also includes also a device 15 for allowing the patient to perform an isometric exercise.

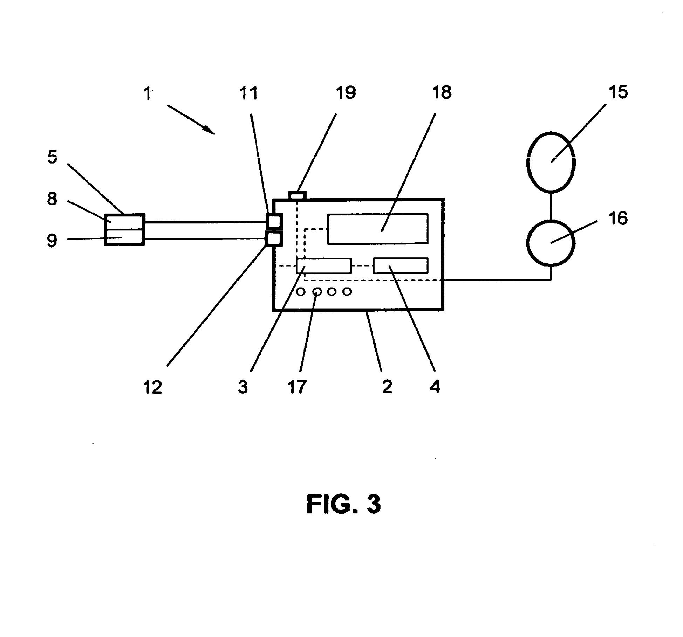

Further, it is possible to derive a phonocardiogram by means of filtering the lower frequencies from the pressure transducer 8. Fig. 3 shows a third embodiment which uses only the combined transducer 5 including the echo probe 8 and pressure sensor 9. The signal from the pressure sensor 9 is filtered so as to obtain both, the higher frequencies in a range of ca. 50 to 500 cps (Hz) representing the phono signal and the lower frequencies of 0.2 to 50 cps (Hz) representing the pressure signal. In this case the transducer can be regarded as a combined echo-presso-phono transducer. Without filtering, it would be impossible to verify the phono-signal because of the significantly higher amplitudes of the presso-signal. It is also possible to slightly shift the crossover frequency between the phono signal and the presso signal towards approx. 100 cps (Hz).

The embodiments as shown in Fig. 1 to 4 are merely illustrative and are not meant to be an exhaustive list of all possible designs, implementations, modifications, and uses of the invention. Moreover, features described in connection with one embodiment of the invention may be used in conjunction with other embodiments, even if not explicitly stated above.

On the basis of the echocardiogram and pressocardiogram obtained by the aforementioned apparatus new combined echo-presso parameters of diastolic function are generated enabling the evaluation of the test result and potentially differentiation of characteristic patterns of diastolic behavior of left ventricle with exercise. Preferably, calculation is performed by means of a computer implemented program on the basis of the combined signals.

The following new combined diastolic indices are introduced by the inventor:

1) Presso-TDI Filling Index = A/H/[E/E'];
2) Presso-transmitral flow A wave index = (A/H)/A;
3) LV end-diastolic Stiffness Index = A/H / LVEDV ;
4) Presso-echo Relaxation Index = TORET(I)/E ;
5) Relaxation Time corrected for ESV = TORET / LVESV(I);
6) Post-Isovolumic Relaxation Time (PIRET) = TORET - IVRT;
7) Post-isovolumic Relaxation Time/Flow index = (TORET-IVRT)/E;
8) Diastolic Combined Relaxation/Compliance Index = [(A/H)/A]/(E/TORET);
9) Global Presso-TDI diastolic Index= (E/E')/DATI;
10) Left atrial index = A/H / (S2/D);
11) Left atrial index in pts with decreased LVEF = A/H /S2;
12) Presso-A/AR index = A/H x AR;
13) Presso-AR duration Index =A/H x AR duration;

wherein for the pressocardiogram: A/H = A-wave amplitude/total pressocardiographic excursion, TORET = total pressocardiographic relaxation time, TORETI = heart rate corrected TORET index, DATI = Diastolic amplitude time index (given by dividing the TORETI with the relative A wave to diastolic deflection, and for Dcppler echocardiogram: E = early diastolic transmitral flow velocity, E' = early diastolic annulus velocity, A = late transmitral flow velocity, LVEDV = left ventricular end-diastolic volume, LVESV = end-systolic volume, IVRT = isovolumic relaxation time, S2 = pulmonary venous second systolic wave, D = pulmonary venous early diastolic wave, AR = pulmonary venous atrial reversed flow wave.

In the signal processor of the interpretation unit 4, there is categorization for example of diastolic types or differential forms being defined partly by use of the mentioned characteristic parameters. Based on the exactly defined limits as well as on some characteristic forms of behavior of the mentioned positivity criteria with exercise, an automated categorization results by proper selection of the most similar diastolic differentialform; this can be outputted to a suitable display which may be a screen 18 or a printed display. Using the instrument with the programmed processing unit 4, an automated categorization can be based on the critical values of mentioned parameters and the direction or magnitude of changes with exercise; thus, establishing a basis for accurate comparison of each patient's data with a known class of patients, whereas a direct status output is produced. The categorization criteria are included in the interpretation unit 4.

The new defined parameters allow for discrimination and subsequent classification as follows:

- Detection of at least one diastolic parameter which is in a range which will be defined as abnormal or pathologic;

- Discrimination of severity of diastolic dysfunction (slight/moderate/severe)

- Discrimination between early (Relaxation) and late (compliance) or combined early and late diastolic abnormalities

- Potential discrimination between Ischemic and Non-ischemic diastolic reaction with isometric exercise.

- Potential differentiation of Differential forms (Hypertensive, Coronary, Cardiomyopathic, Congestive, Advanced)

This classification will be based in a combination of various single and above combined pressure-volume parameters used as exactly defined criteria of abnormality as well as potentially of some exactly defined behavior patterns - including eventually directional changes - and some additional patient's data.

A further classification consists using either separately or at best combined echo-presso indices early and late diastolic abnormalities by defining the "diastolic types":

- early diastolic abnormality (Relaxation or R-type),
- late diastolic abnormality (Compliance or C-type), and
- both C- and R- types are simultaneously present in one single beat (mixed or RC-type)

More particularly, the present invention is suggesting a potentially discrimination of myocardial disease patients with exercise-inducible ischemia from those without inducible ischemia; this separation helping potentially greatly in decision making about admission in the hospital and/or indication for performing a coronary angiography:

- an "Ischemic diastolic response" being defined by a significant exercise-induced rise in LV end-diastolic pressure or an analogous increase in the relative A wave/total excursion of the pressocardiogram or E/E' or one of the combined echo-presso diastolic indices; this alteration being expected to be 50 -75% of the baseline value at rest or/and abnormal absolute or relative (heart rate corrected) prolongation of the relaxation time (TORET in presso- or IVRT or one of the combined echo-presso indices of relaxation),
- a "Non-ischemic" diastolic reaction being defined by the presence of a positive echo-presso stress test in absence of the mentioned ischemic criteria.

A further advantage of development of the present invention consists in at best portable and handheld instrumentation which includes the appropriate computer-based automation of Presso Test and Doppler echocardiographic criteria and categories enabling us to evaluate a patient not only in a hospital, but also at the office or in sports centers and even at his home.

More advantages of the developments in the present invention are given in the claims.

Thus, the result of the present invention is a user friendly small system which does not require expertise physicians - like the classical stress echocardiography utilizing wall motion abnormalities and can be executed in almost every patient (including disabled and elderly) providing useful information about diastolic left ventricular function changes at rest and during isometric exercise.

This system should be ideally automated and could be used widely in the routine clinical practice as initial screening tool for early detection and evaluation of unknown-subclinic or known myocardial disease states including CAD. As a handheld equipment could, it can be applied at every practitioner's office and in outpatient clinics well as in sports centers. Further, the application of this method by such an instrument in daily practice represents a fast, safe and convenient diagnostic modality for every patient.

In contrast, with the present invention using the "combined echo-presso diastolic exercise test" by means of "Handgrip-presso- and echocardiography" - at best portable/handheld - instrument, a rapid and pathophysiologically correct estimation of diastolic LV function using both P & V indices can be performed quickly and noninvasively at every cardiologist's office, at outpatient clinic (incl. CPUs) and even at patient's home by a handheld machine, both at rest and during isometric exercise. A simple readout can provide exact information about early and late diastolic abnormalities within few minutes.

The diagnosis of coronary artery disease or other myocardial diseases based on symptoms is not accurate enough, since these symptoms occur rather late in the course of most myocardial diseases and atypical and above not infrequently entirely absent. Alternatively, ECG and systolic abnormalities are usually preceding the onset of symptoms. However, LV diastolic dysfunction represents the earliest manifestation in the course of most myocardial diseases and the LV diastolic behavior with exercise characterizes some of them.

The probably greatest problem in cardiology but also in health care is still the early identification of subclinic myocardial diseases and especially of asymptomatic patients with CAD. Most practitioners are still based on the daily application of resting ECG or stress ECG or resting Echocardiography neither of which is sensitive enough for myocardial disease detection - especially in the earliest asymptomatic stage. Alternatively, imaging techniques are more sensitive and specific methods such as nuclear (Thallium) imaging or Dobutamine or Treadmill stress echocardiography as well as CT and MRI coronary angiography are time-consuming and are usually used with specific indications. Further, dynamic exercise or pharmacological stress tests are not convenient and not completely safe for the patients; resulting either to a decreased desire of the patients or even contraindication for performing them. Therefore, these techniques can not be applied very widely in office-based practice and often not at outpatient facilities.

A wider application of diastolic tools and especially of "diastolic stress test" using isometric HG exercise as diagnostic modality of early myocardial function alterations, appear to represent an alternative useful approach. Hitherto, the classical diastolic tools, which are widely accepted for detecting LV diastolic dysfunction are Doppler-echocardiography and nuclear techniques at rest as well as more recently magnetic resonance imaging techniques. In addition, although the gold standard of assessing LV diastolic function are still the internally derived LV pressure curve measurements, all of the mentioned current diastolic techniques are assessing the volumetric filling profile -and this mainly at rest- and none is using a pressure transducer.

Thus, although there is no doubt about the existing and significantly increasing interest on and the clinical importance of LV diastolic function assessment, the clinical application of diastolic techniques in the daily practice remains rather limited.

In contrast, a "Combined diastolic stress Echo-Pressocardiography" using HG exercise and an external Tipmanometer, would improve considerably our diagnostic and screening power and enable the detection of subclinic myocardial disease in a faster, easier and convenient way with an expected higher sensitivity and specificity in almost every patient and with a simple read out rather than expert interpretation, with the present invention. Most importantly, this "Combined Echo-Presso HG Stress Test" at best using a handheld/portable equipment could be obtained almost everywhere.

Especially, a low level and Isometric stress test of short duration can be widely applied, independently from physical ability, age and health state; i.e. also in disabled and extreme obese persons can be applied safely and in a convenient way in every patient, since it is of short duration and the exercise mode is only a low level isometric handgrip performed by the one hand with the patient being recumbent on the bed. Therefore, this low level stress test can be widely applied, independently from the level of physical ability, age and health state of the patient.

The technique consists in obtaining simultaneously from the chest wall by means of a "combined echo-presso transducer", comprising both an external pressure transducer and an echo probe, a so-called pressocardiogram and a Doppler-echocardiogram in order to detect LV diastolic dysfunction at rest and during short low level exercise; when needed a dynamic and pharmacological stress test could also be obtained. Specifically, this invention discloses a combined echo-pressocardiographic device - ideally portable/handheld - using a combined echo-presso transducer applicable on the thoracic wall, which enables the assessment and calculation of diastolic LV function indices as well as potentially evaluation of the test results and differentiation of characteristic patterns of diastolic behavior of left ventricle with exercise.

**Claims**

1. Device for noninvasive measurement of parameters of diastolic function of left ventricle, comprising:

   - a combined transducer (5) that is adapted for being applied on the thoracic wall, said transducer including an echo probe (8) and a pressure sensor (9) for obtaining simultaneously signals for Doppler-echocardiograms and pressocardiograms and
   - a calculation and interpretation unit connected with the transducer (5) for receiving and processing said signals.

2. Device according to claim 1, **characterized in that** the calculation unit (2) has an input port (11) for receiving signals from said echo probe (8) and a further input port (12) for receiving signals from said pressure sensor (9).

3. Device according to claim 1 or 2, **characterized in that** the calculation unit (2) has output means for displaying Doppler-echocardiograms, pressocardiograms, and/or parameters derived from a combination of said signals and/or diagrams.

4. Device according to one of claims 1 to 3, **characterized in that** the combined transducer (5) and the calculation and interpretation unit form a handheld portable unit.

5. Device according to one of claims 1 to 4, **character-**

**ized in that** combined transducer (5) comprises a housing (10) and the echo probe (8) and/or the pressure sensor (9) are detachably mounted on the housing.

6. Device according to one of claims 1 to 4, **characterized in that** echo probe (8) and the pressure sensor (9) are integrated in a part of a housing (10), with both parts of the housing (10) being detachably connected with each other.

7. Device according to one of claims 1 to 6, further comprising a heart sounds microphone (6) that is connectable to the calculation and interpretation unit.

8. Device according to one of claims **1** to 6, **characterized in that** the calculation and interpretation unit comprises means for filtering the signals received from the pressure sensor (9) of the combined transducer (5) to obtain a pressure signal and a phono signal.

9. Method of noninvasive measurement of parameters of diastolic function of left ventricle, comprising:

   - obtaining , using the device of any of claims 1-8, simultaneously signals for generating Doppler-echocardiograms and pressocardiograms,
   - generating on the basis of the combined signals echo-presso parameters for describing the diastolic function of the left ventricle.

10. Method according to claim 9, wherein one or several echo-presso parameters are defined as

   1) Presso-TDI Filling Index = A/H/[E/E']; and/or
   2) Presso-transmitral flow A wave index = (A/H)/A; and/or
   3) LV end-diastolic Stiffness Index = A/H / LVEDV ; and/or
   4) Presso-echo Relaxation Index = TORET(I)/E ; and/or
   5) Relaxation Time corrected for ESV = TORET / LVESV; and/or
   6) Post-Isovolumic Relaxation Time (PIRET) = TORET - IVRT; and/or
   7) Post-isovolumic Relaxation Time/Flow index = (TORET-IVRT)/E; and/or
   8) Diastolic Combined Relaxation/Compliance Index = [(A/H)/A]/(E/TORET); and/or
   9) Global Presso-TDI diastolic Index = (E/E')/DATI; and/or
   10) Left atrial index = A/H / (S2/D); and/or
   11) Left atrial index in pts with decreased LVEF = A/H /S2; and/or
   12) Presso-A/AR index = A/H x AR; and/or
   13) Presso-AR duration Index = A/H x AR duration,

wherein for the pressocardiogram: A/H = A-wave amplitude/total pressocardiographic excursion, TORET = total pressocardiographic relaxation time, TORETI = heart rate corrected TORET index, DATI = Diastolic amplitude time index (given by dividing the TORETI with the relative A wave to diastolic deflection, and for Doppler echocardiogram: E = early diastolic transmitral flow velocity, E' = early diastolic annulus velocity, A = late transmitral flow velocity, LVEDV = left ventricular end-diastolic volume, LVESV = end-systolic volume, IVRT= isovolumic relaxation time,

S2 = pulmonary venous second systolic wave, D = pulmonary venous early diastolic wave, AR = pulmonary venous atrial reversed flow wave.

**Patentansprüche**

1.  Gerät für die nichtinvasive Messung von Parametern der diastolischen Funktion des linken Ventrikels, umfassend:

    - einen kombinierten Signalwandler (5), der dazu eingerichtet ist, an der Brustwand angewendet zu werden, wobei der Signalwandler eine Echosonde (8) und einen Drucksensor (9) aufweist, um gleichzeitig Signale für Doppler-Echokardiogramme und Pressokardiogramme zu erstellen, und
    - eine Berechnungs- und Auswertungseinheit, die an den Signalwandler (5) angeschlossen ist, um die Signale zu empfangen und zu verarbeiten.

2.  Gerät nach Anspruch 1, **dadurch gekennzeichnet, dass** die Berechnungseinheit (2) einen Eingangskanal (11) zum Empfangen der Signale von der Echosonde (8) und einen weiteren Eingangskanal (12) zum Empfangen der Signale von dem Drucksensor (9) aufweist.

3.  Gerät nach Anspruch 1 oder 2, **dadurch gekennzeichnet, dass** die Berechnungseinheit (2) eine Ausgabevorrichtung zum Anzeigen der Doppler-Echokardiogramme, Pressokardiogramme und/oder Parameter aufweist, die aus einer Kombination der genannten Signale und/oder Diagramme abgeleitet werden.

4.  Vorrichtung nach einem der Ansprüche 1 bis 3, **dadurch gekennzeichnet, dass** der Signalwandler (5) und die Berechnungs- und Auswertungseinheit eine tragbare, in der Hand zu haltende Einheit bilden.

5.  Gerät nach einem der Ansprüche 1 bis 4, **dadurch gekennzeichnet, dass** der kombinierte Signalwandler (5) ein Gehäuse (10) aufweist und die Echosonde (8) und/oder der Drucksensor (9) lösbar an dem Gehäuse montiert sind.

6.  Gerät nach einem der Ansprüche 1 bis 4, **dadurch gekennzeichnet, dass** die Echosonde (8) und der Drucksensor (9) in einen Teil des Gehäuses (10) integriert sind, wobei beide Teile des Gehäuses (10) lösbar miteinander verbunden sind.

7.  Gerät nach einem der Ansprüche 1 bis 6, weiterhin umfassend ein Herztonmikrophon (6), das an die Berechnungs- und Auswertungseinheit anschließbar ist.

8.  Gerät nach einem der Ansprüche 1 bis 6, **dadurch gekennzeichnet, dass** die Berechnungs - und Auswertungseinheit eine Vorrichtung zum Filtern der vom Drucksensor (9) des kombinierten Signalwandlers (5) empfangenen Signale aufweist, um ein Drucksignal und ein Audiosignal zu ermitteln.

9.  Verfahren für die nichtinvasive Messung von Parametern der diastolischen Funktion des linken Ventrikels, umfassend:

    - Ermitteln zeitgleicher Signale, unter Verwendung des Geräts nach einem der Ansprüche 1 bis 8, um Doppler-Echokardiogramme und Pressokardiogramme zu erzeugen,
    - Erzeugen von Echo-Druck-Parametern auf Basis der kombinierten Signale, um die diastolische Funktion des linken Ventrikels zu beschreiben.

10. Verfahren nach Anspruch 9, wobei ein oder mehrere Echo-Druck-Parameter definiert sind als

    1) Druck-TDI-Füllungsindex = A/H/[E/E']; und/oder
    2) Wellenindex des presso-transmitralen Flusses A = (A/H)/A; und/oder
    3) LV enddiastolischer Steifigkeitsindex = A/H / LVEDV; und/oder
    4) Druck-Echo-Relaxationsindex = TORET(I)/E; und/oder
    5) Relaxationszeit, korrigiert für ESV = TORET / LVESV; und/oder
    6) Post-isovolumetrische Relaxationszeit (PIRET) = TORET - IVRT; und/oder
    7) Post-isovolumetrische Relaxationszeit/Flussindex = (TORET - IVRT)/E; und/oder
    8) Diastolischer kombinierter Relaxations-/Compliance-Index = [(A/H)/A]/(E/TORET); und/oder
    9) Globaler diastolischer Druck-TDI-Index = (E/E')/DATI; und/oder
    10) Linker atrialer Index = A/H / (S2/D); und/oder

11) Linker atrialer Index in Punkten mit abgeschwächter LVEF = A/H/S2; und/oder

12) Druck-A/AR-Index = A/H x AR; und/oder

13) Druck-AR-Dauerindex = A/H x AR Dauer, wobei für das Pressokardiogramm: A/H = A-Wellenamplitude/gesamter pressokardiographischer Ausschlag, TORET = gesamte pressokardiographische Relaxationszeit, TORETI = herzfrequenzkorrigierter TORET-Index, DATI = Diastolischer Amplituden-Zeit-Index (ergibt sich durch Teilen des TORETI durch die relative A-Welle zur diastolischen Ausschlaghöhe), und für das Doppler-Echokardiogramm: E= frühdiastolische transmitrale Flussgeschwindigkeit, E' = frühdiastolische Anulusgeschwindigkeit, A = späte transmitrale Flussgeschwindigkeit, LVEDV = linksventrikuläres enddiastolisches Volumen, LVESV = endsystolisches Volumen, IVRT = isovolumetrische Relaxationszeit, S2 = pulmonal-venöse zweite systolische Welle, D = pulmonal-venöse frühe diastolische Welle, AR = pulmonal-venöse atrial umgekehrte Flusswelle.

## Revendications

1. Dispositif pour la mesure non invasive de la fonction diastolique du ventricule gauche comprenant :

   - un transducteur combiné (5) qui est adapté pour être appliqué sur la paroi thoracique, ledit transducteur comprenant une sonde d'échocardiographie (8) et un capteur de pression (9) pour obtenir simultanément des signaux pour des échocardiogrammes Doppler et des cardiogrammes de pression et
   - une unité de calcul et d'interprétation reliée au transducteur (5) pour recevoir et traiter lesdits signaux.

2. Dispositif selon la revendication 1, **caractérisé en ce que** l'unité de calcul (2) a un port d'entrée (11) pour recevoir des signaux de ladite sonde d'échocardiographie (8) et un autre port d'entrée (12) pour recevoir des signaux dudit capteur de pression (9).

3. Dispositif selon la revendication 1 ou 2, **caractérisé en ce que** l'unité de calcul (2) a des moyens de sortie pour afficher les échocardiogrammes Doppler, les cardiogrammes de pression et/ou les paramètres dérivés d'une combinaison desdits signaux et/ou diagrammes.

4. Dispositif selon l'une des revendications 1 à 3, **caractérisé en ce que** le transducteur combiné (5) et l'unité de calcul et d'interprétation forment une unité portable à la main.

5. Dispositif selon l'une des revendications 1 à 4, **caractérisé en ce que** le transducteur combiné (5) comprend un boîtier (10) et la sonde d'échocardiographie (8) et/ou le capteur de pression (9) sont montés de manière détachable sur le boîtier.

6. Dispositif selon l'une des revendications 1 à 4, **caractérisé en ce que** la sonde d'échocardiographie (8) et/ou le capteur de pression (9) sont intégrés dans une partie d'un boîtier (10) avec les deux parties du boîtier (10) reliées de manière détachables l'une à l'autre.

7. Dispositif selon l'une des revendications 1 à 6 comprenant de plus un microphone pour les sons cardiaques (6) qui peut être relié à l'unité de calcul et d'interprétation.

8. Dispositif selon l'une des revendications 1 à 6, **caractérisé en ce que** l'unité de calcul et d'interprétation comprend des moyens pour filtrer les signaux reçus du capteur de pression (9) du transducteur combiné (5) pour obtenir un signal de pression et un signal du phono.

9. Procédé de mesure non invasive de paramètres de la fonction diastolique du ventricule gauche comprenant :

   - l'obtention, en utilisant le dispositif selon l'une quelconque des revendications 1 à 8, simultanément de signaux pour générer des échocardiogrammes Doppler et des cardiogrammes de pression,
   - la génération sur la base des paramètres des signaux combinés d'échocardiogramme et de pression pour décrire la fonction diastolique du ventricule gauche.

10. Procédé selon la revendication 9 dans lequel un ou plusieurs paramètres échocardiogramme et pression sont définis en tant que :

   1) indice de remplissage TDI pression = A/H/[E/E'] et/ou
   2) indice d'onde A du flux transmitral pression = (A/H)/A et/ou
   3) indice de rigidité de fin de diastole ventricule gauche = A/H/LVEDV et/ou
   4) indice de relaxation échographie pression = TORET(I)/E et/ou
   5) temps de relaxation corrigé pour ESV = TORET / LVESV et/ou
   6) temps de relaxation post-isovolumique (PIRET) = TORET- IVRT et/ou
   7) temps de relaxation post-isovolumique/indice de flux = (TORET-IVRT)/E et/ou
   8) indice de relaxation combinée diastolique/compliance = [(A/H)/A](E/TORET) et/ou

9) indice diastolique global TDI pression = (E/E')
DATI et/ou

10) indice oreillette gauche = A/H/(S2/D) et/ou

11) indice oreillette gauche en points avec LVEF
diminuée = A/H/S2 et/ou

12) indice pression A/AR = A/H x AR et/ou

13) indice de durée pression AR = A/H x durée
AR

dans lequel pour le cardiogramme de pression :
A/H = amplitude onde A/excursion de cardiogramme de pression totale, TORET = temps de
relaxation pression cardiogramme total, TORETI = indice TORET corrigé de vitesse du coeur,
DAI = indice de temps d'amplitude diastolique
(donné en divisant le TORETI par l'onde A relative à la déflexion diastolique et pour l'échocardiogramme Doppler : E = vitesse de flux
transmitral précoce, LVEDV = volume de fin de
diastole du ventricule gauche, LVESV = volume
de fin de systole, IVRT = temps de relaxation
isovolumique, S2 = onde systolique seconde
veineuse pulmonaire, D = onde diastolique précoce veineuse pulmonaire, AR = onde de flux
retour d'oreillette veineux pulmonaire.

**FIG. 1**

**Fig. 2**

**FIG. 3**

**FIG. 4**

0.1s

**FIG. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7107095 B2

- DE 19740931 C2

### Non-patent literature cited in the description

- **DR. MANOLAS.** 1st European Diastology Meeting 1996 and at the 1st Annual Meeting of the Heart Failure Society of America 1997 as well as in Clin. Cardiol. *Identification of Patients with Coronary Artery Disease by Assesing Diastolic Abnormities during Isometric Exercise,* 2001, vol. 24, 735-743
- **A. P. FLESSAS et al.** Effects of isometric exercise on the end-diastolic pressure, volumes, and function of the left ventricle in man. *Circulation,* 1976, vol. 53, 839-847
- **B. D. JOHNSON et al.** Persistent chest pain predicts cardiovascular events in women without obstructive coronary artery disease: results from the NIH-NHLBI-sponsored Women's Ischaemia Syndrome Evaluation (WISE) study. *European Heart Journal,* 2006, vol. 27 (12), 1408-1415

- **U. SIGWART et al.** Left atrial function in acute transient left ventricular ischemia produced during percutaneous transluminal coronary angioplasty of the left anterior descending coronary artery. *The American Journal of Cardiology,* 1990, vol. 65 (5), 282-286
- **M. I. BURGESS et al.** Diastolic Stress Echocardiography: Hemodynamic Validation and Clinical Significance of Estimation of Ventricular Filling Pressure With Exercise. *Journal of the American College of Cardiology,* 2006, vol. 47 (9), 1891-1900